# EUROPÄISCHE PATENTSCHRIFT

(11) **EP 3 525 779 B1**
(45) Veröffentlichungstag und Bekanntmachung des Hinweises auf die Patenterteilung: **05.06.2024**
(21) Anmeldenummer: 17777915.4
(22) Anmeldetag: 05.10.2017
(51) Int. Cl.: A61P 11/00, A61P 13/12, A61P 43/00, A61K 31/167, A61K 31/506, A61K 45/06

(54) **KOMBINATION ENTHALTEND DEN SGC STIMULATOR VERICUGUAT UND DEN MINERALCORTICOID-REZEPTOR-ANTAGONIST FINERENONE**
COMBINATION CONTAINING THE SGC STIMULATOR VERICUGUAT AND THE MINERALCORTICOID RECEPTOR ANTAGONIST FINERENONE
COMBINAISON CONTENANT LE STIMULATEUR SGC VERICUGAT ET L'ANTAGONISTE DES RÉCEPTEURS MINÉRALCORTICOÏDES FINERENONE

(30) Priorität: 11.10.2016 EP 16193364
(43) Veröffentlichungstag der Anmeldung: 21.08.2019
(73) Patentinhaber: Bayer Pharma Aktiengesellschaft, 13353 Berlin (DE)
(72) Erfinder: KOLKHOF, Peter, 42113 Wuppertal (DE); SANDNER, Peter, 42113 Wuppertal (DE)
(74) Vertreter: BIP Patents
(86) Internationale Anmeldenummer: PCT/EP2017/075286
(87) Internationale Veröffentlichungsnummer: WO 2018/069126

(56) Entgegenhaltungen:
- WO-A1-2011/161099
- DE-A1-102007 009 494
- DE-A1-102010 021 637
- HAO A. TRAN ET AL: "Potential new drug treatments for congestive heart failure", EXPERT OPINION ON INVESTIGATIONAL DRUGS, Bd. 25, Nr. 7, 17. Mai 2016 (2016-05-17), Seiten 811-826, XP055425494, UK ISSN: 1354-3784, DOI: 10.1080/13543784.2016.1181749
- FUTATSUGI KENTARO ET AL: "Design and synthesis of aryl sulfonamide-based nonsteroidal mineralocorticoid receptor antagonists", BIOORGANIC & MEDICINAL CHEMISTRY LETTERS, Bd. 23, Nr. 23, 8. Oktober 2013 (2013-10-08), Seiten 6239-6242, XP028760183, ISSN: 0960-894X, DOI: 10.1016/J.BMCL.2013.09.099

## Beschreibung

Die vorliegende Erfindung betrifft eine Kombination, die den sGC Stimulator Vericiguat (Methyl-{4,6-diamin-2- [5 -fluor-1 -(2-fluorbenzyl)- 1H-pyrazolo [3,4-b]pyridin-3 -yl]pyrimidin-5 -yl} carbamat der Formel (X) 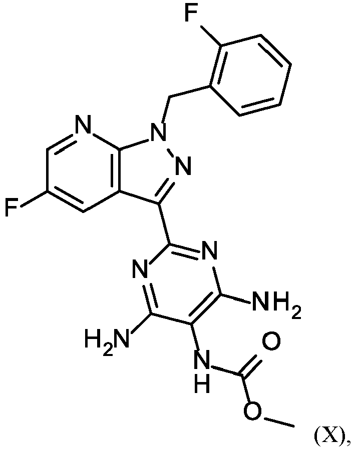 und Finerenone (*S*)-4-(4-Cyano-2-methoxyphenyl)-5-ethoxy-2,8-dimethyl-1,4-dihydro-1,6-naphthyridin- 3-carboxamid der Formel (IV) 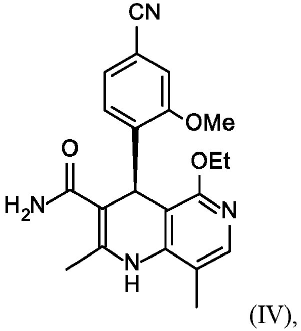 enthält, sowie die Verwendung der Kombination zur Behandlung und/oder Prophylaxe von Herz- und Herz-Kreislauf-Erkrankungen, von Nieren- und kardio-renalen Erkrankungen, von Lungen- und kardio-pulmonalen Erkrankungen, sowie zur Behandlung und/oder Prophylaxe von fibrotischen Erkrankungen.

Die gesamte Körperfunktion von Mensch und Tier wird durch Regelkreisläufe kontrolliert und aufrechterhalten. Bestandteile dieser physiologischen Regelkreisläufe sind wiederum Kaskadensysteme körpereigener Hormone, Enzyme und Rezeptoren. Diese Regelkreisläufe sind miteinander verbunden und werden zentral gesteuert. Pathophysiologische Veränderungen innerhalb des Körpers, aber auch Einflüsse von außen wie z.B. Klima, Stress, Nahrungsbestandteile einschließlich Medikamente, haben direkte Einflüsse auf diese Regelkreisläufe. Dadurch verminderte oder überschießende Aktivitäten einzelner Komponenten dieser Kaskaden und Regelsysteme können durch Gegensteuerung mittels sogenannter Rückkopplungs- (,feed-back`) oder auch Vorwärtskopplungs- (,feed-forward') Mechanismen ausgeglichen werden. Eine kurzfristige Gegensteuerung, z.B. durch eine kompensatorische Freisetzung eines bestimmten körpereigenen Hormons ist somit für die Aufrechterhaltung der Körperfunktion in Notsituationen (z.B. bei Verletzungen) lebenswichtig. Allerdings kann auch eine permanente kompensatorische Gegenregulation langfristig fatale Folgen für den Gesamtorganismus haben.

Die meisten Therapieansätze zur Behandlung von Erkrankungen des Herz- und Herzkreislaufsystems, Nieren- und kardio-renalen oder des Lungen- und kardio-pulmonären Systems und fibrotischen Erkrankungen greifen in eines der angesprochenen Regelsysteme ein. Dies kann den Nachteil haben, dass aufgrund einer kompensatorischen Gegenregulation des Körpers bereits nach kurzer Zeit eine Desensitierung erfolgt und die gewünschte Wirkung weniger oder nicht mehr erzielt wird und dadurch u.a. höhere Dosierungen verwendet werden müssen. Damit gehen Nachteile einher, wie bspw. ein höheres Nebenwirkungspotential.

Die Aufgabe der vorliegenden Erfindung besteht in der Bereitstellung von Kombinationen pharmazeutisch aktiver Substanzen, die in mehr als einen Regelkreislauf einwirken und die zur Behandlung von Erkrankungen des Herz- und Herzkreislaufsystems, Nieren- und kardio-renalen Systems oder des Lungen- und kardio-pulmonalen Systems und fibrotischen Erkrankungen verwendet werden können.

Eines dieser oben genannten essentiellen Regelsysteme ist das sogenannte Renin-Angiotensin-Aldosteron-System (RAAS). Es ist ein zentrales Kaskadensystem von Hormonen und Enzymen, die den Salz- und Wasserhaushalt und damit den Blutdruck des Körpers steuern. Durch Salz- und Flüssigkeitsmangel oder Blutdruckabfälle wird in speziellen Nierenzellen das Hormon Renin gebildet und ausgeschüttet. Renin spaltet das in der Leber gebildete Angiotensinogen in Angiotensin I, während das Angiotensin Conversions-Enzym (ACE) Angiotensin I in Angiotensin II umwandelt. Angiotensin II besitzt potente gefäßverengende und damit blutdrucksteigernde Wirkungen und stimuliert die Bildung des Steroidhormons Aldosteron in der Nebennierenrinde. Aldosteron fördert die Rückaufnahme von Natrium aus dem Urin ins Blut, wodurch das Blutvolumen steigt.

Die spezifischen Effekte von Angiotensin II werden über entsprechende extrazelluläre Rezeptoren (Angiotensin-Rezeptor, AT-R) vermittelt, die auf Zielzellen des kardiovaskulären Systems exprimiert werden. Dagegen werden die spezifischen Effekte von Aldosteron über einen intrazellulären Rezeptor, dem Aldosteron- oder Mineralocorticoid-Rezeptor (MR) vermittelt. Neben ihrer zentralen Bedeutung bei der Salz-, Wasser- und Blutdruckregulation besitzen sowohl Angiotensin II als auch Aldosteron direkte pro-inflammatorische und -fibrotische Eigenschaften. Beide Hormone spielen insbesondere bei Umbauprozessen (,remodeling`) in Herz, Nieren und Gefäßen, die z.B. durch einen Herzinfarkt oder ein akutes Nierenversagen induziert werden, eine wesentliche Rolle: So stimuliert z.B. Aldosteron die Einlagerung von Kollagenproteinen im Herzmuskel, was zu einer erhöhten Steifigkeit und damit langfristig zu einer verminderten Funktionalität führen kann.

Aldosteron und Angiotensin II bilden einen klassischen ,feed-forward` Regelkreis: Neben Kalium ist Angiotensin II der wichtigste Stimulus zur Freisetzung von Aldosteron aus der Nebenniere und umgekehrt stimuliert Aldosteron in Herzgewebe und Gefäßen die Produktion von ACE, also dem Enzym, das aus dem Vorläufer-Angiotensin das Angiotensin II generiert. Die pathopysiologischen Wirkungen von Angiotensin II und Aldosteron können durch entsprechende Inhibitoren der ACE, des AT-R und des MR vermindert werden, jedoch unterliegen diese singulären Blockaden den o.g. ,feed-back` Kompensationsmechanismen, d.h. Blockade des Mineralocorticoid-Rezeptors führt zu einer kompensatorischen Freisetzung von Aldosteron, ähnlich wie AT-R Blockade zu einem Anstieg von Angiotensin II führt. Langfristige Kompensationsmechanismen spielen beim klinisch bedeutsamen ,Aldosterone Escape` Phänomen eine besondere Rolle: Da die Aldosteron-Freisetzung sozusagen der letzte Schritt in der RAAS-Kaskade darstellt, war man lange Zeit der Meinung, dass die Blockade der initialen RAAS-Schlüsselschritte wie der ACE-Aktivität oder des AT-Rs ausreichen, um auch den letzten Schritt, die Aldosteronbildung und -freisetzung in der Nebenniere zu verhindern. Allerdings zeigte die RESOLVD Studie (McKelvie et al., Circulation 1999;100;1056-1064), dass sowohl unter singulärer ACE oder AT-R Blockade, als auch unter dualer ACE/AT-R Blockade die Aldosteron-Plasmaspiegel bei Patienten mit Herzinsuffizienz zwar innerhalb der ersten 17 Behandlungs-Wochen gegenüber dem Ausgangswert vermindert wird, allerdings nach 43 Wochen den Ausgangswert sogar übersteigt. Die Ergebnisse dieser Studie belegen, dass eine Verhinderung der Bindung von Aldosteron an den Mineralocorticoid-Rezeptor zusätzlich zur Angiotensin Blockade klinisch enorm bedeutsam ist.

MR Antagonisten (wie z.B. die steroidalen Verbindungen Spironolactone, Canrenone/Canrenoat und Eplerenone, sowie neuere nicht-steroidale MR Antagonisten wie z.B. MT-3995 gemäß der Verbindung der Formel (VI), CS-3150 gemäß der Verbindung der Formel (V), LY2623091, PF-03882845 gemäß der Verbindung der Formel (XXXII) 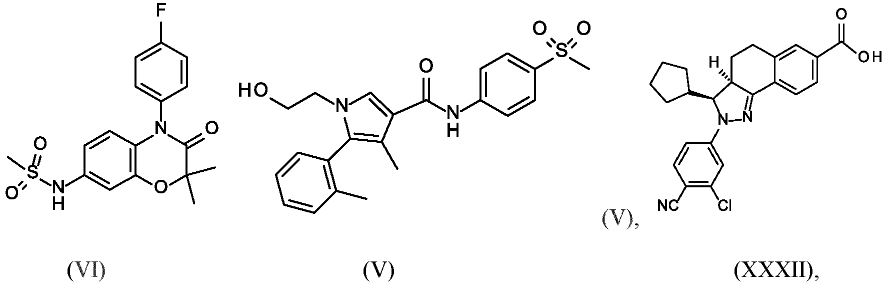 und Finerenone, gemäß der Verbindung der Formel (IV)) wirken der, durch Aldosteron vermittelten Natriumretention in den Nieren entgegen (natriuretische Wirkung).

Somit führen MR Antagonisten zu einer vermehrten Natriumausscheidung, was bei hypertensiven Patienten und/oder bei Patienten mit Herzinsuffizienz und/oder Niereninsuffizienz ein bewährtes Therapiekonzept darstellt. Allerdings können MR Antagonisten ihre natriuretische Wirkung nur in den Nierensegmenten entfalten, in denen Aldosteron auch über den MR physiologisch wirksam ist. Das sind v.a. die spät distalen Tubulus- und Sammelrohrabschnitte, die nur zu einem begrenzten Teil an der Natriumrückresorption beteiligt sind, während der Hauptanteil der Natriumsekretion und - rückresorption in proximalen Tubulusabschnitten abläuft.

Neben dem RAAS gibt es aber auch noch andere sehr wichtige Regelsysteme und eines davon ist der Stickstoffmonoxid (Nitric Oxide/NO) cyclische Guanosinmonophosphat (cGMP) und Phosphodiesterase (PDE) Signalweg (NO/cGMP Weg). Durch Blutdruckerhöhung und daraus resultierende Schärkräfte auf die Endothelzellen in Blutgefässen wird durch Enzyme in den Endothelzellen aber auch in Nervenendigungen, den sogenannten NO-Synthasen (NOS), aus L-Arginin das NO gebildet. Dieses NO ist gasförmig und diffundiert über die Zellmembranen hinweg in verschiedene Zielzellen, insbesondere zu den Gefäßmuskelzellen. Dort bindet es an die Hämgruppe in der löslichen Guanylatzyklase (sGC), einem heterodimeren, aus einer alpha- und einer beta-Untereinheit bestehenden, intrazellulären Hämprotein. Die NO-Bindung aktiviert das Enzym, welches dann die Umwandlung von Guanosin-Triphosphat (GTP) in cGMP katalysiert. Dieses cGMP stellt ein wichtiges sogenanntes "Second Messenger" Molekül dar und bindet an eine Vielzahl von intrazellulären Proteinen, unter anderem die cGMP-abhängien Proteinkinasen (G-Kinasen). G-Kinasen vermitteln über Phosphorylierung verschiedener Zielproteine, z.B. von Kaliumkanälen, eine Senkung des intrazellulären Kalziumspiegels, wodurch z.B. eine Relaxation der Gefäßmuskulatur ausgelöst wird. Daher wirkt NO über eine Stimulation des sGC/cGMP Weges gefäßdilatierend und damit blutdrucksenkend. Darüber hinaus sind für cGMP auch zahlreiche andere Wirkungen, wie z.B. eine anti-thrombotische, anti-fibrotische oder anti-inflammatorische Wirkung, beschrieben worden. Diese Wirkungen sind allerdings molekular weniger gut verstanden als die Gefäßdilatation und noch nicht vollständig aufgeklärt. Die NO/cGMP Signalkaskade und die Wirkungen von cGMP werden durch Abbau des cGMP zu unwirksamen GMP beendet. Dieser Schritt, die Hydrolyse des zyklischen Phosphatringes und die Bildung von 5 GMP wird durch Phosphodiesterasen (PDE) katalysiert. Bei den PDEs handelt es sich um eine große Familie von Enzymen mit bisher elf identifizierten Mitgliedern und über 100 verschiedene sogenannten Splicingvarianten. Die verschiedenen PDEs unterscheiden sich vor allem hinsichtlich der Gewebespezifität (z.B. PDE6 wird ausschließlich im Auge exprimiert), der Substratspezifität (z.B. cAMP- oder cGMP-spezifisch) und der Regulation (z.B. durch Kalzium/Calmodulin oder zyklische Nukleotide). Für die spezifische Spaltung von cGMP sind vor allem die PDEs vom Typ 5 (PDE5), 6 (PDE6) und 9 (PDE9) verantwortlich, (vgl. Übersichtsarbeiten zum NO/cGMP/PDE Signalweg, z.B. Conti & Beavo 2007, Schmidt et al. (Herausgeber) 2009; Stasch et al. 2011, Derbyshire and Marletta 2012, Monica et al. 2016)

In Anbetracht der großen Bedeutung des NO/cGMP Signalweges für die physiologische Regulation und Aufrechterhaltung von Körperfunktionen, insbesondere für die Funktion des Herz- und Herz-Kreislaufsystemes, des Gefäßsystems, der Nierenfunktion oder der Lungen und kardio-pulmonären Funktion, aber auch von anitifibrotischen Effekten, wurden eine Reihe von Pharmaka erforscht und entwickelt die an verschiedenen wichtigen Umschaltstellen in diesen Signalweg eingreifen. Dies war umso mehr notwendig, da bekannt ist, dass verschieden Erkrankungen der oben genannten Organsysteme mit einer verminderten Bildung von NO einhergehen, was zu einer unzureichenden cGMP Versorgung führt und eine der zugrunden liegenden Pathomechanismen bei der Entwicklung von Herz- und Herz-Kreislauf, Nieren-, Lungen- und fibrotischen Erkrankungen sein könnte.

Seit langem bekannt ist z.B. die Verwendung von Nitraten und NO-Donoren, die bei der Behandlung von Angina Pectoris, sowohl zur akuten Anfallskupierung als auch bei der Anfallsprophylaxe, eingesetzt werden. Diese Verbindungen setzten enzymatisch oder nicht-enzymatisch NO frei, das dann an die sGC binden kann und zur cGMP Erhöhung führt. Allerdings stellen neben den kinetischen Limitationen dieser Verbindungen, vor allem eine erhöhte Radikalbildung die gefäß- und organschädigend wirken können, sowie die Entwicklung einer Tachyphylaxie eine signifikante Einschränkung des therapeutischen Potentials dar.

Daher fokussierten sich neuere Entwicklungen unter anderem auf die Hemmung des cGMP Abbaus durch die Hemmung von spezifischen PDEs, insbesondere die Hemmung der PDE5. Die Entwicklung von potenten und selektiven PDE5 Inhibitoren, wie z.B. Sildenafil, Vardenafil oder Tadalafil, zeigten erneut die Wirksamkeit dieses Signalweges für die Regulation des Gefäßtonus. Es erfolgten klinische Zulassungen von Präparaten für die Behandlung von erektiler Dysfunktion (ED), von pulmonaler arterieller Hypertonie (PAH) und von benigner Prostatahyperaplasie (BPH). Außerdem werden diese Verbindungen auch für den Einsatz bei Herz- und Herz-Kreislauferkrankungen und bei Nierenerkrankungen klinisch erprobt. Dies zeigt erneut die ubiquitäre Bedeutung dieses NO/cGMP Signalweges und unterstreicht das breite Anwendungspotential dieser cGMP erhöhenden Verbindungen. Die Behandlungsmöglichkeiten mit PDE5 Inhibitoren sind allerdings beschränkt, da diese für Ihre Wirksamkeit einen ausreichend hohen endogenen cGMP Spiegel benötigen, der durch die Verbindungen dann vor Abbau geschützt ist. In vielen Erkrankungen, insbesondere auch in Herz- und Herzkreislauferkrankungen oder Lungenerkrankungen ist aber die endogene NO-Produktion und damit auch die cGMP Bildung zumindest teilweise gestört. Daher wirken PDE5 Inhibitoren auch nicht in allen Patienten vergleichsweise gut bzw. gibt es auch behandlungsresistente Patienten, z.B. bei der erektilen Dysfunktion oder bei pulmonaler Hypertonie.

Um diese Limitation sowohl von Nitraten wie von PDE5 Inhibitoren zu umgehen wurde versucht die sGC direkt pharmakologisch zu stimulieren. Dies sollte zum Einen die NO-abhängige Radikalbildung der Nitrate, zum Anderen aber auch die Abhängigkeit der Wirksamkeit vom produzierten cGMP, wie sie für PDE5 Inhibitoren beschrieben ist, umgehen. Die Erforschung und Entwicklung von sGC Stimulatoren und sGC Aktivatoren stellt daher einen Meilenstein in der Pharmakologie des NO/cGMP Signalweges dar. Diese beiden Verbindungsklassen, sGC Stimulatoren und sGC Aktivatoren, stimulieren die sGC NO unabhängig und führen zu einer NO-unabhängigen Produktion von cGMP. Daneben wirken aber diese Verbindungsklassen auch synergistisch (sGC Stimulatoren) und additiv (sGC Aktivatoren) zum endogen gebildeten NO. Der Hauptunterschied, soweit dies momentan bekannt ist, besteht in der Bindung an die sGC. Die sGC ist ein heterodimeres Protein, das aus einer alpha- und einer beta-Untereinheit, letzter trägt die NO-bindende Hämgruppe, gebildet wird. Die sGC Stimulatoren binden an die alpha Untereinheit der nicht oxidierten und hämhaltigen sGC und bewirken die direkte, NO unabhängige Bildung von cGMP (Stasch et al. (A) 2001; Stasch & Hobbs 2009). Die sGC Aktivatoren binden hingegen an die beta-Untereinheit der oxidierten und hämfreien sGC, aktivieren diese und führen so zur NO-unabhängigen Bildung von cGMP (Stasch et al. (B) 2001, Schmidt et al. 2009). Obgleich dieser prinzipielle Unterschied sehr gut unter in vitro Bedingungen etabliert ist, sind die physiologischen und pathophysiologischen Konsequenzen des Vorkommens von hämhaltiger und oxidierter-hämfreier sGC und das sich daraus ableitende Behandlungspotential dieser Verbindungsklassen noch unvollständig verstanden. Dennoch wurde der pharmakologische Nutzen von sGC Stimulatoren und sGC Aktivatoren in zahlreichen präklinischen Modellen und für zahlreiche verschiedene Indikationen, insbesondere im Bereich von Herz- und Herz-Kreislauf-Erkrankungen, von Nieren- und kardio-renalen Erkrankungen, von Lungen- und kardio-pulmonären Erkrankungen gezeigt (Evgenov et al. 2009, Stasch et al. 2011). Dies konnte auch in klinischen Studien bestätigt werden und so wurde im Jahre 2013 der sGC Stimulator Riociguat für die Behandlung von pulmonaler arterieller Hypertonie (PAH) und von chronisch thrombo-embolischer pulmonaler Hypertonie (CTEPH) zugelassen (Ghofrani et al. (A), Ghofrani et al. (B) 2013, 2013Hambly & Granton 2015). Zudem befindet sich der sGC Stimulator Vericiguat in Phase II/III für die Behandlung von Herzinsuffizienz (Pieske et al. 2014, Gheorghiade et al. 2015). Diese Beispiele belegen auch klinisch die breite Einsatzmöglichkeit von sGC Stimulatoren und sGC Aktivatoren, im Bereich der Behandlung und/oder Prophylaxe von Herz- und Herz-Kreislauf-Erkrankungen, von Nieren- und kardio-renalen Erkrankungen bzw. von Lungen- und kardio-pulmonären Erkrankungen. Zudem konnte präklinisch sehr gut eine anti-fibrotische Wirkung von sGC Stimulatoren und sGC Aktivatoren gezeigt werden.

Grundsätzlich unterscheidet man MR Antagonisten (MRAs) mit einer steroidalen Grundstruktur von solchen, die über einen nicht-steroidale Grundstruktur verfügen. Steroidale MRAs wie Spironolakton oder dessen aktiver Metabolit Kanrenone interagieren nicht nur mit dem MR, sondern auch mit den homologen Androgen und Progesteron Rezeptoren. Diese Interaktionen können zu unerwünschten Wirkungen auf den Sexualhormonstoffwechsel wie Gynecomastie, Dysmenorrhoe und Libidoverlust führen. Nicht-steroidale MRAs wie Finerenone interagieren spezifisch mit dem MR, so dass entsprechende Nebenwirkungen, die aus Interaktionen mit anderen Steroidhormonrezeptoren resultieren können, nicht zu erwarten sind.

In der erfindungsmäßen Kombination wird Finerenone verwendet.

Finereneone ((*S*)-4-(4-Cyano-2-methoxyphenyl)-5-ethoxy-2,8-dimethyl-1,4-dihydro-1,6-naphthyridin-3-carboxamid), ist ein selektiver Antagonist, der auf einem Dihydropyridingrundgerüst basiert, der Formel (IV) 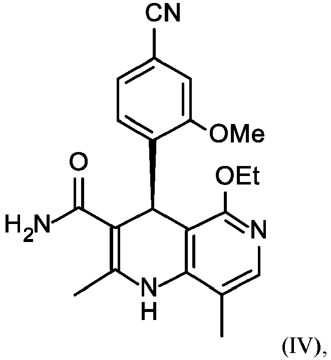 welches in der DE 10 2007009494 A1 und WO 2008 104 306 A2 beschrieben ist,

In der erfindungsgemäßen Kombination wird der sGC Stimulator Vericiguat eingesetzt:
Vericiguat (Methyl-{4,6-diamin-2-[5-fluor-1-(2-fluorbenzyl)-1H-pyrazolo[3,4-b]pyridin-3-yl]pyrimidin-5-yl}carbamat der Formel (X) 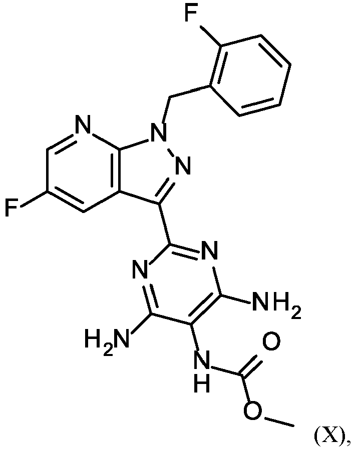 welches in der WO2011/147809 offenbart ist,

Die Verwendung substituierte 5-Fluor-1H-Pyrazolopyridine allein oder in Kombinationen zur Behandlung und/oder Prophylaxe von Herz-Kreislauf- Erkrankungen wird in DE 102010021637 A1 genannt.

Sowohl das RAAS wie der NO/cGMP Weg spielen eine wichtige Rolle bei der Aufrechterhaltung der Körperhomeostase und regeln wichtige Funktionen im Herz- und Herzkreislaufsystem, Nieren- und kardio-renalen System oder im Lungen und kardio-pulmonären System. Die Verwendung von MR-Antagonisten sowie die Verwendung von sGC Stimulatoren, als Monotherapien bei Herz- und Herz-Kreislauf-Erkrankungen, bei Nieren- und kardio-renalen Erkrankungen, bei Lungen- und kardio-pulmonären Erkrankungen oder bei fibrotischen Erkrankungen ist ebenfalls beschrieben. Tatsächlich ist aber sowohl der Anteil der Dysregulation der beiden Signalwege bei diesen verschiedenen Erkrankungen, als auch die absolute Effizienz der beiden Mechanismen im direkten Vergleich unbekannt.

Daher wurden Versuche durchgeführt um sGC Stimulatoren mit MR-Antagonisten direkt zu vergleichen. Dadurch sollten Aufschlüsse über die qualitative- und quantitative Krankheitsrelevanz dieser beiden Pathomechanismen und die sich daraus ableitbaren Behandlungsparadigmen erforscht werden.

Es wurden in diesen Versuchen Kombinationen von MR-Antagonisten mit sGC Stimulatoren eingesetzt und auch im direkten Vergleich zu den Einzelverbindungen getestet. Überraschend zeigte sich eine außergewöhnliche Wirksamkeit dieser Kombinationen die weit über die Wirksamkeit der Einzelkomponenten hinaus geht und eine synergistische Wirkung der Kombination aus MR-Antagonisten und sGC Stimulatoren nahe legt.

Die Lösung der oben gestellten Aufgabe kann somit in der Bereitstellung von Kombinationen gesehen werden, die mindestens einen sGC Stimulator und mindestens einen Stoff, der in das RAAS-System eingreift enthalten, und die zur gezielten Behandlung von Herz- und Herz-Kreislauf-Erkrankungen, Nieren- und kardio-renalen Erkrankungen, Lungen- und kardio-pulmonären Erkrankungen und fibrotischen Erkrankungen mit über-additiven Effekten verwendet werden können.

Die Erfindung stellt daher u.a. eine Kombination, die den sGC Stimulator Vericiguat (Methyl-{4,6-diamin-2- [5 -fluor-1-(2-fluorbenzyl)-1H-pyrazolo [3,4-b]pyridin-3 -yl]pyrimidin-5 -yl} carbamat der Formel (X) 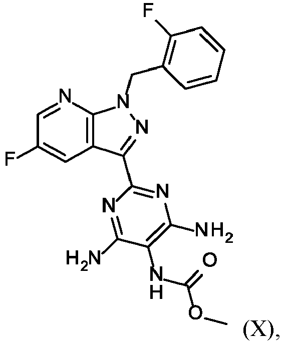 und Finerenone (*S*)-4-(4-Cyano-2-methoxyphenyl)-5-ethoxy-2,8-dimethyl-1,4-dihydro-1,6-naphthyridin- 3-carboxamid der Formel (IV) 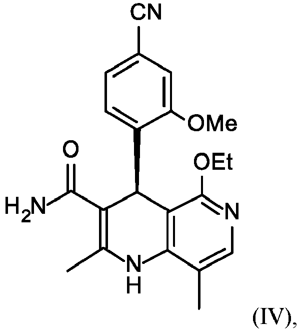 enthält, bereit.

Die Kombination zeigt unter akutem und insbesondere unter chronischem Einsatz, positive Effekte bzgl. Endorganschutz von Herzen und Nieren, Verminderung der renalen Proteinausscheidung, Verminderung von Morbidität und Mortalität unter experimentellen Bedingungen. Diese experimentellen Bedingungen bestehen zum einen aus gesunden Tieren oder auch Tieren mit Bluthochdruck, Herz- und/oder Niereninsuffizienz (z.B. transgene Renin-Ratten), L-NAME behandelten Tieren (z.B. transgene Renin-Ratten + L-NAME Behandlung).

Die gleichzeitige Blockade der Bindung von Aldosteron an MR und der Aktivierung der löslichen Guanylatcyclase durch sGC Stimulatoren durch die erfindungsgemäße Kombination, führt zu überadditiven Effekten bzgl. des Endorgan-Schutz, der Verminderung der renalen Proteinausscheidung und Verminderung von Morbidität und Mortalität.

Ein weiterer Gegenstand der Erfindung ist die medizinische Verwendung der Kombinationen, die den sGC Stimulator Vericiguat (Methyl-{4,6-diamin-2- [5-fluor-1-(2-fluorbenzyl)-1H-pyrazolo[3,4-b]pyridin-3-yl]pyrimidin-5-yl}carbamat der Formel (X) 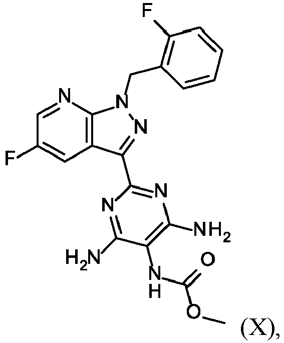 und Finerenone (S)-4-(4-Cyano-2-methoxyphenyl)-5-ethoxy-2,8-dimethyl-1,4-dihydro-1,6-naphthyridin- 3-carboxamid der Formel (IV) 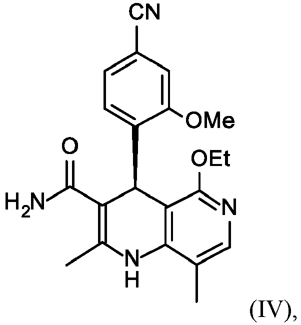 enthält. In einer Ausführungsform dient diese Kombination zur Behandlung und/oder Prophylaxe von Krankheiten.

Eine weitere Ausführungsform der Erfindung ist die Verwendung der erfindungsgemäßen Kombination in einem Verfahren zur Behandlung und/oder Prophylaxe von Herz-, Herz-Kreislauf-Erkrankungen, Nieren-, kardio-renalen Erkrankungen, Lungen- und kardio-pulmonalen Erkrankungen, sowie zur Behandlung und/oder Prophylaxe von fibrotischen Erkrankungen sowie von Diabetes und diabetischer und nicht-diabetischer Retinopathie.

Eine weitere Ausführungsform der Erfindung ist die Behandlung und/oder Prophylaxe vonHerzinsuffizienz mit erhaltener Ejektionsfraktion oder Herzinsuffizienz mit reduzierter Ejektionsfraktion, Behandlung und/oder Prophylaxe von Vorhofflimmern, Schlaganfall oder Atherosklerose, renalen und kardio-renalen Erkrankungen, chronisches Nierenversagen oder diabetische Nephropathie.

Eine weitere Ausführungsform der Erfindung ist die Behandlung und/oder Prophylaxe von Bluthochdruck, Herzinsuffizienz (akut und chronisch), dekompensierter Herzinsuffizienz, linksventrikulärer Dysfunktion, hypertrophischer Kardiomyopathie, diabetischer Kardiomyopathie, supraventrikulären und ventrikulären Arrythmien, Vorhofflimmern, Vorhofflattern, schädlichem Gefäßumbau, Herzinfarkt und Folgeerscheinungen davon, Atherosklerose, Angina (instabil oder stabil), Niereninsuffizienz (diabetisch und nichtdiabetisch), Herzinsuffizienz, Angina pektoris, Diabetes, sekundärem Hyperaldosteronismus, primärer und sekundärer pulmonaler Hypertonie, Glomerulonephritis, Sklerodermie und Systemischer Sklerose, glomulerärer Sklerose, Proteinurie als Folge einer primären Nierenkrankheit, renaler vaskulärer Hypertonie, diabetischer und nicht-diabetischer Retinopathie, Migräne, periphärer Gefäßkrankheit, Raynaud-Krankheit, luminaler Hyperplasie, kognitiver Dysfunktion, Glaukom und Schlaganfall.

Bevorzugt ist die Anwendung von der erfindungsgemäßen Kombination zur Behandlung von Herz- und Herz-Kreislauf-Erkrankungen wie z.B. Herzinsuffizienz mit erhaltener Ejektionsfraktion oder Herzinsuffizienz mit reduzierter Ejektionsfraktion, Behandlung und/oder Prophylaxe von Vorhofflimmern, Schlaganfall oder Atherosklerose, zur Behandlung von renalen und cardio-renalen Erkrankungen wie z.B. chronisches Nierenversagen oder diabetische Nephropathie, von Lungenerkrankungen und cardio-pulmonären Erkrankungen, wie z.B. von Pulmonaler Hypertonie, Erkrankungen des Zentralnervensystems, zur Behandlung und/oder Prophylaxe von fibrotischen Erkrankungen sowie anderen Krankheitserscheinungen (z.B. Endorganschäden, die Hirn, Niere, Herz oder Lunge betreffen).

Ein weiterer erfindungsgemäßer Gegenstand der Erfindung ist ein Arzneimittel enthaltend mindestens eine Kombination, die den sGC Stimulator Vericiguat (Methyl-{4,6-diamin-2- [5-fluor-1-(2-fluorbenzyl)-1H-pyrazolo[3,4-b]pyridin-3-yl]pyrimidin-5-yl}carbamat der Formel (X) 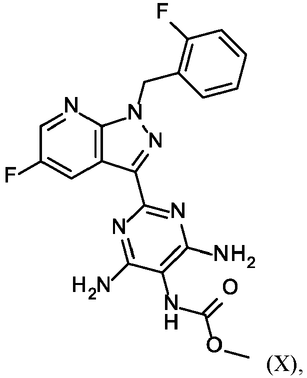 und Finerenone (S)-4-(4-Cyano-2-methoxyphenyl)-5-ethoxy-2,8-dimethyl-1,4-dihydro-1,6-naphthyridin- 3-carboxamid der Formel (IV) 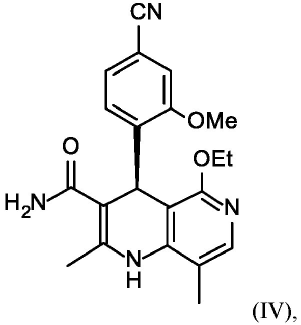 enthält, in Kombination mit einem inerten, nicht-toxischen, pharmazeutisch geeigneten Hilfsstoff.

Ein Gegenstand der vorliegenden Erfindung ist eine pharmazeutische Formulierung enthaltend eine Kombination von Finerenone (S)-4-(4-Cyano-2-methoxyphenyl)-5-ethoxy-2,8-dimethyl-1,4-dihydro-1,6-naphthyridin- 3-carboxamid der Formel (IV) und dem sGC Stimulator Vericiguat (Methyl-{4,6-diamin-2- [5-fluor-1-(2-fluorbenzyl)- 1H-pyrazolo [3,4-b]pyridin-3 -yl]pyrimidin-5 -yl} carbamat der Formel (X), sowie die Salze, Solvate und Solvate der Salze der zu kombinierenden Komponenten.

Die zu kombinierenden Komponenten können als Salze vorliegen. Als Salze sind im Rahmen der vorliegenden Erfindung physiologisch unbedenkliche Salze der zu kombinierenden Verbindungen bevorzugt. Umfasst sind auch Salze, die für pharmazeutische Anwendungen selbst nicht geeignet sind, jedoch beispielsweise für die Isolierung oder Reinigung der zu kombinierenden Verbindungen verwendet werden können.

Die erfindungsgemäßen Kombinationen ist geeignet für die Prophylaxe und/oder Behandlung von verschiedenen Erkrankungen und krankheitsbedingten Zuständen, insbesondere zur Behandlung und/oder Prophylaxe von Herz- und Herz-Kreislauf-Erkrankungen wie z.B. Herzinsuffizienz mit erhaltener Ejektionsfraktion oder Herzinsuffizienz mit reduzierter Ejektionsfraktion, Behandlung und/oder Prophylaxe von Vorhofflimmern, Schlaganfall oder Atherosklerose, renalen und kardio-renalen Erkrankungen wie z.B. chronisches Nierenversagen oder diabetische Nephropathie, Lungenerkrankungen und kardiopulmonäre wie z.B. von Pulmonaler Hypertonie, Erkrankungen des Zentralnervensystems, zur Behandlung und/oder Prophylaxe von fibrotischen Erkrankungen sowie anderen Krankheitserscheinungen (z.B. Endorganschäden, die Hirn, Niere oder Herz betreffen).

Weiterhin sind die erfindungsgemäßen Kombinationen geeignet für die Prophylaxe und/oder Behandlung von verschiedenen Erkrankungen und krankheitsbedingten Zuständen, insbesondere zur Behandlung und/oder Prophylaxe von einer Krankheit ausgewählt aus der Gruppe bestehend aus Bluthochdruck, Herzinsuffizienz (akut und chronisch), dekompensierter Herzinsuffizienz, linksventrikulärer Dysfunktion, hypertrophischer Kardiomyopathie, diabetischer Kardiomyopathie, supraventrikulären und ventrikulären Arrythmien, Vorhofflimmern, Vorhofflattern, schädlichem Gefäßumbau, Herzinfarkt und Folgeerscheinungen davon, Atherosklerose, Angina (instabil oder stabil), Niereninsuffizienz (diabetisch und nichtdiabetisch), Herzinsuffizienz, Angina pektoris, Diabetes, sekundärem Hyperaldosteronismus, primärer und sekundärer pulmonaler Hypertonie, Glomerulonephritis, Sklerodermie und Systemischer Sklerose, glomulerärer Sklerose, Proteinurie als Folge einer primären Nierenkrankheit, renaler vaskulärer Hypertonie, diabetischer und nicht-diabetischer Retinopathie, Migräne, periphärer Gefäßkrankheit, Raynaud-Krankheit, luminaler Hyperplasie, kognitiver Dysfunktion, Glaukom und Schlaganfall.

Weiterer Gegenstand der vorliegenden Erfindung ist die Verwendung der erfindungsgemäßen Kombinationen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen.

Die erfindungsgemäßen Kombinationen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Kombinationen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- den Blutdruck senkende Wirkstoffe, beispielhaft und vorzugsweise aus der Gruppe der Calcium-Antagonisten, Angiotensin Rezeptor Blocker (ARBs), ACE-Hemmer, Endothelin-Antagonisten, Renin-Inhibitoren, alpha-Rezeptoren-Blocker, beta-Rezeptoren-Blocker und Rho-Kinase-Inhibitoren;
- Diuretika, insbesondere Schleifendiuretika sowie Thiazide und Thiazid-ähnliche Diuretika;
- Antidiabetika, beispielhaft und vorzugsweise Insulin und Derivate, Sulfonharnstoffe, Biguanide, Thiazolidinedione, Acarbose, DPP4 Inhibitoren, GLP-1 Analoge, oder SGLT Inhibitoren (Gliflozine);
- antithrombotisch wirkende Mittel, beispielhaft und vorzugsweise aus der Gruppe der Thrombozytenaggregationshemmer, der Antikoagulantien oder der profibrinolytischen Substanzen;
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugs-weise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten;
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- positiv-inotrop wirksame Verbindungen, wie beispielsweise Herzglycoside (Digoxin), betaadrenerge und dopaminerge Agonisten wie Isoproterenol, Adrenalin, Noradrenalin, Dopamin und Dobutamin;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil, sowie PDE 3-Inhibitoren wie Amrinone und Milrinone;
- natriuretische Peptide, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Hemmstoffe der Endopeptidasen (NEP-Inhibitoren) wie z.B. Sacubitril, Omapatrilat oder AVE-7688, oder in dualer Kombination ('ARNIs') mit Angiotensin Rezeptor Blockern (z.B. Valsartan), wie z.B. LCZ696,
- Calcium-Sensitizer, wie beispielhaft und vorzugsweise Levosimendan;
- If-Kanal-Blocker, wie beispielhaft und vorzugsweise Ivabradin;
- Myosin Aktivatoren, wie beispielhaft und vorzugsweise Omecamtiv mecarbil;
- Inhibitoren der humanen neutrophilen Elastase (HNE), wie beispielsweise Sivelestat oder DX-890 (Reltran);
- die Signaltransduktionskaskade inhibierende Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussende Verbindungen, wie beispielhaft und vorzugsweise Etomoxir, Dichloracetat, Ranolazine oder Trimetazidine.

Bei einer bevorzugten Ausführungsform der Erfindung wird die erfindungsgemäße Kombination in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Blutdruck senkenden Mitteln werden vorzugsweise Verbindungen aus der Gruppe der Calcium-Antagonisten, Angiotensin Rezeptor Blocker, ACE-Hemmer, Endothelin-Antagonisten, Renin-Inhibitoren, alpha-Rezeptoren-Blocker, beta-Rezeptoren-Blocker, Rho-Kinase-Inhibitoren sowie der Diuretika verstanden.

Bei einer bevorzugten Ausführungsform der Erfindung wird das erfindungsgemäße Kombinationspräparat in Kombination mit einem Antidiabetikum, wie beispielhaft und vorzugsweise Insulin und Derivate, Sulfonylharnstoffe wie Tolbutamid, Carbutamid, Acetohexamid, Chlorpropamid, Glipizid, Gliclazid, Glibenclamid, Glyburid, Glibomurid, Gliquidon, Glisoxepid, Glyclopyramid, Glimepirid, JB253 und JB558, Meglitinide wie Repaglinid und Nateglinid, Biguanide wie Metformin und Buformin, Thiazolidinedione wie Rosiglitazone und Pioglitazone, Alpha-Glucosidase Inhibitoren wie Miglitol, Acarbose und Voglibose, DPP4 Inhibitoren wie Vildagliptin, Sitagliptin, Saxagliptin, Linagliptin, Alogliptin, Septagliptin und Teneligliptin, GLP-1 Analoge wie Exenatid (auch Exendin-4), Liraglutid, Lixisenatid und Taspoglutid, oder SGLT Inhibitoren (Gliflozine) wie Canagliflozin, Dapagliflozin und Empagliflozin.

Bei einer bevorzugten Ausführungsform der Erfindung wird die erfindungsgemäße Kombination in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugs-weise Losartan, Candesartan, Valsartan, Olmesartan, Telmisartan oder Embursatan, verabreicht.

Bei einer bevorzugten Ausführungsform der Erfindung wird die erfindungsgemäße Kombination in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung wird die erfindungsgemäße Kombination in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung wird die erfindungsgemäße Kombinationt in Kombination mit einem Renin-Inhibitor, wie beispielhaft und vorzugsweise Aliskiren, SPP-600, SPP-635, SPP-676, SPP-800 oder SPP-1148, verabreicht.

Bei einer bevorzugten Ausführungsform der Erfindung wird die erfindungsgemäße Kombination in Kombination mit einem alpha-1-Rezeptoren-Blocker, wie beispielhaft und vorzugsweise Prazosin, verabreicht.

Bei einer bevorzugten Ausführungsform der Erfindung wird die erfindungsgemäße Kombination in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung wird die erfindungsgemäße Kombination in Kombination mit einem Rho-Kinase-Inhibitor, wie beispielhaft und vorzugsweise Fasudil, Y-27632, SLx-2119, BF-66851, BF-66852, BF-66853, KI-23095 oder BA-1049, verabreicht.

Bei einer bevorzugten Ausführungsform der Erfindung wird die erfindungsgemäße Kombinationt in Kombination mit Prostanoiden und Prostacyclinrezeptor Agonisten, wie beispielhaft und vorzugsweise Iloprost, Beraprost, Cicaprost, Epoprostenol oder Treprostinil.

Unter antithrombotisch wirkenden Mitteln (Antithrombotika) werden vorzugsweise Zusammensetzungen aus der Gruppe der Thrombozytenaggregationshemmer, der Antikoagulantien oder der profibrinolytischen Substanzen verstanden.

Bei einer bevorzugten Ausführungsform der Erfindung wird die erfindungsgemäße Kombination in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung wird die erfindungsgemäße Kombination in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung wird die erfindungsgemäße Kombination in Kombination mit einem GPIIb/IIIa-Antagonisten, wie beispielhaft und vorzugsweise Tirofiban oder Abciximab, verabreicht.

Bei einer bevorzugten Ausführungsform der Erfindung wird die erfindungsgemäß Kombination in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung wird die erfindungsgemäße Kombination in Kombination mit Heparin oder einem low molecular weight (LMW)-Heparin-Derivat verabreicht.

Bei einer bevorzugten Ausführungsform der Erfindung wird die erfindungsgemäße Kombination in Kombination mit einem Vitamin K-Antagonisten, wie beispielhaft und vorzugsweise Coumarin, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung wird die erfindungsgemäße Kombination mit einem CETP-Inhibitor, wie beispielhaft und vorzugsweise Torcetrapib (CP-529 414), JJT-705, BAY 60-5521, BAY 78-7499 oder CETP-vaccine (Avant), verabreicht.

Bei einer bevorzugten Ausführungsform der Erfindung wird die erfindungsgemäße Kombination in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung wird die erfindungsgemäße Kombination in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung wird die erfindungsgemäße Kombination in Kombination mit einem Squalensynthese-Inhibitor, wie beispielhaft und vorzugsweise BMS-188494 oder TAK-475, verabreicht.

Bei einer bevorzugten Ausführungsform der Erfindung wird die erfindungsgemäße Kombination in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung wird die erfindungsgemäße Kombination in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung wird die erfindungsgemäße Kombination in Kombination mit einem PPAR-gamma-Agonisten, wie beispielhaft und vorzugsweise Pioglitazone oder Rosiglitazone, verabreicht.

Bei einer bevorzugten Ausführungsform der Erfindung wird die erfindungsgemäße Kombination mit einem PPAR-delta-Agonisten, wie beispielhaft und vorzugsweise GW-501516 oder BAY 68-5042, verabreicht.

Bei einer bevorzugten Ausführungsform der Erfindung wird die erfindungsgemäße Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung wird die erfindungsgemäße Kombination mit einem Lipase-Inhibitor, wie beispielhaft und vorzugsweise Orlistat, verabreicht.

Bei einer bevorzugten Ausführungsform der Erfindung wird die erfindungsgemäße Kombination in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung wird die erfindungsgemäße Kombination in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung wird die erfindungsgemäße Kombination in Kombination mit einem Lipoprotein(a)-Antagonisten, wie beispielhaft und vorzugsweise Gemcabene calcium (CI-1027) oder Nicotinsäure, verabreicht.

Bei der bevorzugten Ausführungsform der Erfindung wird die erfindungsgemäße Kombination in Kombination mit anti-fibrotisch wirksamen Verbindungen, wie beispielhaft und vorzugsweise Sorafenib, Regorafenib, Imatinib, Dasatinib, Nilotinib Nintedanib, Bortezomib oder Pirfenidone verabreicht.

Bei der bevorzugten Ausführungsform der Erfindung wird die erfindungsgemäße Kombination in Kombination mit anti-inflammatorisch wirksamen Verbindungen wie beispielhaft und vorzugsweise Cyclophosphamide, Methotrexate, Rapamycin, Azathioproin, Tocilizumab, Infliximab, Rituximab, Adalimumab, Belimumab, Abatacept, SAR100842 oder Thalidomide Derivate verabreicht.

Die erfindungsgemäßen Kombinationen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Kombinationen in geeigneten Applikationsformen verabreicht werden.

Für die orale Applikation eignen sich nach dem Stand der Technik funktionierende, die erfindungsgemäßen Kombinationen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Kombinationen kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Als bevorzugte Applikationsformen sind zu nennen Tablettenform (nicht-überzogene oder überzogene Tabletten, beispielsweise mit magensaftresistenten oder sich verzögert auflösenden oder unlöslichen Überzügen, die die Freisetzung der erfindungsgemäßen Kombinationen kontrollieren), in der Mundhöhle schnell zerfallende Tabletten oder Filme/Oblaten.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, wobei die orale Applikation bevorzugter ist. Insbesondere bevorzugt ist die orale Applikation mittels Tablettenform.

Die erfindungsgemäßen Kombinationen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

In den erfindungsgemäßen Kombinationen können die Komponenten zusammen oder nacheinander oder getrennt in einer kombinierten Einheitsdosierungsform oder in zwei getrennten Einheitsdosierungsformen verabreicht werden. Die Einheitsdosierungsform kann auch eine fixierte Kombination sein.

Eine therapeutisch wirksame Menge jeder Komponente der erfindungsgemäßen Kombination kann simultan oder sequenziell in jeder Reihenfolge verabreicht werden.

In einer Ausführungsform können die Komponenten in einer sogenannten Retard-formulierung vorliegen, in der die Freisetzung der erfindungsgemäßen Komponenten zu unterschiedlichen Zeitpunkten stattfindet. Beispielsweise genannt sei eine Tablette mit sich verzögert auflösenden Überzügen, die jeweils eine oder mehrere der erfindungsgemäßen Komponenten enthält.

Für den Fall, dass die Komponenten der erfindungsgemäßen Kombination in getrennten Einheitsdosierungsformen verabreicht werden, können die MR Antagonisten und sGC Stimulatoren beispielsweise jeweils als Kapsel oder Tablette bereitgestellt werden.

Bei oraler Applikation beträgt beispielsweise die Dosierung von Finerenone gemäß der Verbindung der Formel (IV) etwa 1 bis 100 mg od, vorzugsweise etwa 2.5 bis 50 mg od und ganz besonders bevorzugt 10 bis 40 mg od.

Bei oraler Applikation beträgt beispielsweise die Dosierung von Vericiguat gemäß der Verbindung der Formel (X) etwa 0.5 bis 50 mg od, vorzugsweise etwa 1 bis 15 mg od und ganz besonders bevorzugt 1.25 bis 10 mg od.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Erfindung betrifft auch die Kombination von getrennten pharmazeutischen Zusammensetzungen in Kitform. Dies ist ein Kit, der zwei getrennte Einheiten umfasst: Eine pharmazeutische Zusammensetzung umfassend den MR Antagoinst Finerenone und eine pharmazeutische Zusammensetzung umfassend den sGC Stimulator Vericiguat.

Die Erfindung betrifft außerdem eine bevorzugte Kitform, die zwei Einheiten umfasst: Eine pharmazeutische Zusammensetzung enthaltend den MR Antagonisten Finerenone und eine pharmazeutische Zusammensetzung enthaltend den sGC Stimulator Vericiguat.

Das Kit ist insbesondere vorteilhaft, wenn die getrennten Komponenten in unterschiedlichen Dosisformen verabreicht werden müssen oder in unterschiedlichen Dosisintervallen verabreicht werden.

### Ausführungsbeispiele für pharmazeutische Zusammensetzungen

Die erfindungsgemäßen Verbindungen können folgendermaßen in pharmazeutische Zubereitungen überführt werden:

### Tablette:

### Pharmazeutische Formulierung von Finerenone (4S)- 4-(4-Cyano-2-methoxyphenyl)-5-ethoxy-2,8-dimethyl-1,4-dihydro-1,6-naphthyridin-3-carbox-amid der Formel (IV)

Es wurde eine Granulatlösung der Verbindung der Formel (IV) in kristalliner Form in mikronisierter Form, Hypromellose 5 cP, Natriumlaurilsufat in gereinigtem Wasser hergestellt.

Cellulose microcrystalline, lactose monohydrate and croscarmellose sodium wurden in einem Behälter oder einem fluidized bed granulator gemischt (premix).

Der premix und die Granulatlösung wurden in dem fluid-bed granulator granuliert.

Das Lubricant Magnesium Stearat wurde hinzugegeben, nachdem das Granulat getrocknet und gesiebt wurde. Damit wurde eine pressfertige Mischung hergestellt.

Unter Verwendung einer Rotationstablettenpresse wurde die pressfertige Mischung zu Tabletten gepresst.

Eine homogene coating Suspension wurde aus hypromellose, talc, titanium dioxid, Eisenoxid gelb, Eisenoxid rot und gereinigten Wasser hergestellt. In einer geeigneten coating Vorrichtung wurde die coating Suspension auf die Tabletten gesprüht.

| **Zusammensetzung** | **Ph IIb** | **Ph IIb** | **Ph IIb** | **Ph IIb** | **Ph IIb** | **Ph IIb** | **Ph IIb** |
|---|---|---|---|---|---|---|---|
| **Verbindung** | [mg] | [mg] | [mg] | [mg] | [mg] | [mg] | [mg] |
| der Formel (IV) in der mikronisiert | 1.25 | 2.50 | 5.00 | 7.50 | 10.00 | 15.00 | 20.00 |
| **Excipients** | | | | | | | |
| Cellulose microcrystalline | 73.80 | 72.50 | 69.90 | 67.30 | 64.70 | 62.00 | 59.30 |
| Croscarmellose sodium | 4.50 | 4.50 | 4.50 | 4.50 | 4.50 | 4.50 | 4.50 |
| Hypromellose 5 cP | 4.50 | 4.50 | 4.50 | 4.50 | 4.50 | 4.50 | 4.50 |
| Lactose monohydrate | 45.00 | 45.00 | 45.00 | 45.00 | 45.00 | 42.50 | 40.00 |
| Magnesium stearate | 0.90 | 0.90 | 0.90 | 0.90 | 0.90 | 0.90 | 0.90 |
| Sodium laurilsulfate | 0.05 | 0.10 | 0.20 | 0.30 | 0.40 | 0.60 | 0.80 |
| **Gewicht (unbeschichtete Tablette)** | **130.00** | **130.00** | **130.00** | **130.00** | **130.00** | **130.00** | **130.00** |

| **Film-coating** | | | | | | | |
|---|---|---|---|---|---|---|---|
| Hypromellose 5 cP | 3.0336 | 3.0336 | 3.0336 | 3.0336 | 3.0336 | 3.0336 | 3.0336 |
| Titanium dioxid | 2.3196 | 2.3196 | 2.3196 | 2.3196 | 2.3196 | 2.3196 | 2.3196 |
| Talcum | 0.6072 | 0.6072 | 0.6072 | 0.6072 | 0.6072 | 0.6072 | 0.6072 |
| Eisenoxid gelb | 0.0324 | 0.0324 | 0.0324 | 0.0324 | 0.0324 | 0.0324 | 0.0324 |
| Eisenoxid rot | 0.0072 | 0.0072 | 0.0072 | 0.0072 | 0.0072 | 0.0072 | 0.0072 |
| **Gewicht (film-coating)** | **6.0000** | **6.0000** | **6.0000** | **6.0000** | **6.0000** | **6.0000** | **6.0000** |
| **Gewicht (beschichtete Tablette)** | **136.00** | **136.00** | **136.00** | **136.00** | **136.00** | **136.00** | **136.00** |

### Bewertung der physiologischen Wirksamkeit

Die Eignung der erfindungsgemäßen Kombinationen zur Behandlung von Herz- und Herz-Kreislauferkrankungen sowie Nieren- und kardio-renalen Erkrankungen und anderen in der Anmeldung beschriebenen Erkrankungen kann in folgenden Assaysystemen gezeigt werden:

### 1.) In vivo-Test zum Nachweis von natriuretischer Wirksamkeit an wachen Ratten in Stoffwechselkäfigen

Wistar Ratten (250-450 g Körpergewicht) werden mit freiem Zugang zu Futter (Altromin) und Trinkwasser gehalten. Ab ca. 72 Stunden vor Versuchsbeginn erhalten die Tiere anstelle des normalen Futters ausschließlich Kochsalz-reduziertes Futter mit einem Gehalt von 0.02% Natrium-Chlorid (ssniff R/M-H, 10mm mit 0,02% Na, S0602-E081, ssniff Spezialdiäten GmbH, D-59494 Soest). Während des Versuches werden die Tiere für ca. 24 Stunden einzeln in für Ratten dieser Gewichtsklasse geeigneten Stoffwechselkäfigen (Tecniplast Deutschland GmbH, D-82383 Hohenpeißenberg) mit freiem Zugang zu Kochsalz-reduziertem Futter und Trinkwasser gehalten. Am Versuchsbeginn wird den Tieren die zu prüfende Substanz in einem Volumen von 0,5 ml/kg Körpergewicht eines geeigneten Lösemittels mittels einer Schlundsonde in den Magen verabreicht. Als Kontrolle dienende Tiere erhalten nur Lösemittel. Kontrollen und Substanztestungen werden am selben Tag parallel durchgeführt. Kontrollgruppen und Substanzdosisgruppen bestehen aus jeweils 6 bis 8 Tieren. Während des Versuchs wird der von den Tieren ausgeschiedene Urin kontinuierlich in einem Auffangbehälter am Käfigboden gesammelt. Für jedes Tier wird gesondert das Urinvolumen pro Zeiteinheit bestimmt und die Konzentration der im Urin ausgeschiedenen Natrium- bzw. Kalium-Ionen mittels flammenphotometrischer Standardmethoden gemessen. Die Messintervalle betragen typischerweise den Zeitraum bis zu 8 Stunden nach Versuchsbeginn (Tagintervall) und den Zeitraum von 8 bis 24 Stunden nach Versuchsbeginn (Nachtintervall).

### 2.) DOCA/Salz-Modell

Die Verabreichung von Desoxycorticosteronacetat (DOCA) in Kombination mit einer Hochsalzdiät und einseitiger Nierenentfernung bei der Ratte induziert einen Hypertonus, der durch relativ niedrige Reninspiegel charakterisiert ist. Als Folge dieser endokrinen Hypertonie (DOCA ist eine direkte Vorstufe von Aldosteron) kommt es, abhängig von der gewählten DOCA-Konzentration, zu einer Hypertrophie des Herzens und weiteren Endorganschäden, z.B. der Niere, die u.a. durch Proteinurie und Glomerulosklerosis charakterisiert sind. In diesem Rattenmodell lassen sich somit Testsubstanzen auf vorhandene antihypertrophe und endorganschützende Wirkung hin untersuchen.

Etwa 8 Wochen alte (Körpergewicht zwischen 250 und 300 Gramm), männliche Sprague Dawley (SD-) Ratten werden linksseitig uninephrektomiert. Dazu werden die Ratten mit 1.5-2%igem Isofluran in einer Mischung aus 66% N₂O und 33% Oz anästhesiert und die Niere wird über einen Flankenschnitt entfernt. Als spätere Kontrolltiere dienen sogenannte sham operierte Tiere, denen keine Niere entfernt wird.

Uninephrektomierte SD-Ratten erhalten 1% Natriumchlorid im Trinkwasser und einmal wöchentlich eine subkutane Injektion DOCA (Firma SIGMA, gelöst in Sesamöl; Hochdosis: 100 mg/kg/wk s.c.; Normaldosis: 30 mg/kg/wk s.c.) zwischen die Schulterblätter gespritzt.

Die Substanzen, die auf ihre protektive Wirkung in vivo untersucht werden sollen, werden per Gavage oder per Futter verabreicht (Ssniff, Germany). Die Tiere werden einen Tag vor Versuchsbeginn randomisiert und Gruppen mit gleicher Tierzahl, in der Regel n=8-15, zugeordnet. Während des gesamten Versuches steht den Tieren Trinkwasser und Futter ad libitum zur Verfügung. Die Substanzen (Kombinationen) werden einmal täglich 4-12 Wochen lang per Gavage oder per Futter verabreicht. Als Plazebogruppe dienen Tiere, die genauso behandelt werden, aber entweder nur das Lösungsmittel oder das Futter ohne Testsubstanz erhalten.

Am Versuchsende können hämodynamische Parameter (Blutdruck, Herzfrequenz, Inotropie [dp/dt], Relaxationszeit [tau], maximaler linksventrikulärer Druck, linksventrikulärer enddiastolischer Druck [LVEDP]) gemessen werden, sowie die Gewichte von Herz, Niere und Lunget, Proteinausscheidung und Genexpression von Biomarkern (z.B. BNP, Brain Natriuretic Peptide, Plasma Renin Aktivität, Angiotensin und Aldosteron) mittels RIA, ELISA oder RT/TaqMan PCR nach RNA Isolation aus kardialem und renalen Gewebe bestimmt.

### 3.) L-NAME behandelte, transgene Renin-Ratte (TGR(mRen2)27)

Die transgene Renin-Ratte ,TGR(mRen2)27` ist eine, von Mullins und Ganten entwickelte hypertensive Rattenlinie, die das Ren-2-Gen der Maus überexprimiert. Durch zusätzliche Gabe des Stickstoffmonoxid-Synthase-Inhibitors L-NAME induziert man eine endotheliale Dysfunktion, die Morbidität und Mortalität in diesem Modell erhöht. Homozygote Tiere versterben an sekundären Komplikationen wie Herz- und Niereninsuffizienz, oder Schlaganfall, sofern sie keiner lebenslangen, antihypertensiven Therapie unterzogen werden.

Männliche TGR(mRen2)27 Renin-Ratten im Alter von 10 bis 20 Wochen werden bzgl. verschiedenener pharmakologischer Behandlungsgruppen, sowie einer Plazebo-Gruppe randomisiert. Zusätzlich erfolgt die Gabe des Inhibitors der Stickstoffmonoxid-Synthase, L-NAME über das Trinkwasser in einer Konzentration von 30 bis 100 mg/l. Während des gesamten Versuches steht den Tieren Trinkwasser und Futter ad libitum zur Verfügung. Die Substanzen werden täglich 4-10 Wochen lang per Gavage oder per Futter verabreicht. Als Plazebogruppe dienen Tiere, die genauso behandelt werden, aber entweder nur das Lösungsmittel oder das Futter ohne Testsubstanz erhalten. Während des Versuchs erfolgt in regelmäßigen Abständen die Bestimmung des systolischen Blutdrucks per Schwanzmanschette (tail cuff), sowie die Bestimmung von Proteinurie und Urinelektrolytzusammensetzung durch Urinsammlung in metabolischen Käfigen. Am Versuchsende werden hämodynamische Parameter (Blutdruck, Herzfrequenz, Inotropie [dp/dt], Relaxationszeit [tau], maximaler linksventrikulärer Druck, linksventrikulärer enddiastolischer Druck [LVEDP]) gemessen, sowie die Gewichte von Herz, Niere und Lunge bestimmt, Proteinausscheidung und Biomarker (z.B. ANP, RIA Kit RK 005-24, Phoenix Pharmaceuticals, Inc., USA, cGMP, RIA Kit RE29075, IBL International GmbH, Hamburg, Renin, Angiotensin I, RIA Kit CA-1533, DiaSorin S.p.A., Italy, und Aldosterone, P2714, DiaSorin S.p.A., Italy), sowie Genexpression von Biomarkern mittels RT/TaqMan PCR nach RNA Isolation aus kardialem und renalen Gewebe bestimmt.

### Beispiele

Herz- und Herzkreislauferkrankungen wie auch Nieren und kardiorenale Erkrankungen sind von einer hohen Morbidität der Patienten aber auch von einer hohen Mortalität gekennzeichnet. Diese Morbidität und Mortalität zusammen mit verschiedenen Risikofaktoren wie z.B. Bluthochdruck, lässt sich sehr gut im bereits beschriebenen Tiermodell der mit L-NAME behandelten Renin transgenen Ratte abbilden. Daher wurde z.B. dieses Tiermodell verwendet um MR-Antagonisten wie z.B. Finerenone gemäß der Verbindung der Formel (IV) und sGC Stimulatoren, wie z.B. die Verbindung der Formel (X) und Kombinationen aus Beiden zu untersuchen:
Z.B. wurden der MR-Antagonist Finerenone entsprechend der Verbindung der Formel (IV) und der sGC Stimulator entsprechend der Verbindung der Formel (X) alleine und in Kombinationen in TGR(mRen2)27 Renin-Ratten im Alter von 10 bis 20 Wochen getestet. Zusätzlich erfolgte die Gabe des Inhibitors der Stickstoffmonoxid-Synthase, L-NAME über das Trinkwasser in einer Konzentration von 30 bis 100 mg/l. Während des gesamten Versuches standen den Tieren Trinkwasser und Futter ad libitum zur Verfügung. Die Substanzen wurden täglich 4-10 Wochen lang per Gavage verabreicht. Als Plazebogruppe dienten Tiere, die genauso behandelt wurden, aber nur das Lösungsmittel für die Testsubstanz erhalten haben. In der Versuchsserie wurde neben Placebo (Gruppe A), der MR-Antagonist Finerenone entsprechend der Verbindung der Formel (IV) (10mg/kg od) (Gruppe B) und der sGC Stimulator entsprechend der Verbindung der Formel (X) (0.3 mg/kg od) (Gruppe C) alleine, und eine Kombination aus Finerenone entsprechend der Verbindung der Formel (IV) (10mg/kg od) + die Verbindung der Formel (X) (0.3 mg/kg od) (Gruppe D) verabreicht. Pro Gruppe (A, B, C, D,) wurden in dieser Studie 15 Tiere eingesetzt (Tabelle 1):

| **Gruppenbezeichnung** | **Behandlung** | **Dosis** | **Gruppengröße [n]** |
|---|---|---|---|
| Gruppe A | Placebo | | 15 |
| Gruppe B | Finerenone, (Verbindung der Formel (IV)) | 10 mg/kg od | 15 |
| Gruppe C | Verbindung der Formel (X) | 0.3 mg/kg od | 15 |
| Gruppe D | Finerenone (Verbindung der Formel (IV)) + Verbindung der Formel (X) | 10 mg/kg od + 0.3 mg/kg od | 15 |

Tabelle 1: Gruppeneinteilung, Behandlung, eingesetzte Dosierung und Applikationsschema (od/once daily = einmal täglich; bid/bidaily = zweimal täglich) und Gruppengröße der mit L-NAME behandelten Renin transgenen Ratten.

### Mortalität:

Nachdem 40% der Placebotiere verstorben waren - der Studienabbruch erfolgte in der Regel wenn 40-50% der mit Placebo behandelten Tiere verstorben sind was einer Überlebensrate von 60-50% entspricht - wurde die Studie abgebrochen und die Überlebensraten in den einzelnen Behandlungsgruppen miteinander verglichen. Es zeigte sich, dass mit einer Behandlung von Finerenone entsprechend der Verbindung der Formel (IV) (10 mg/kg od) alleine bzw. der sGC Stimulator entsprechend der Verbindung der Formel (X) (0.3 mg/kg od) alleine, nur 20% bzw. 13% der Tiere im Studienzeitraum verstarben was Überlebensraten von 80% bzw. 87% entspricht. Allerdings verhinderte eine Kombination aus Finerenone entsprechend der Verbindung der Formel (IV) (10 mg/kg od) + Verbindung der Formel (X) (0.3 mg/kg od) alle Todesfälle über den Studienzeitraum hinweg und führte zu eine signifikanten höheren Überleben mit einer Überlebensrate von100% im Studienzeitraum (Tabelle 2):

**Tabelle 2: Gesamtmortalität ("all cause mortality") während des Studienzeitraumes der mit L-NAME behandelten Renin transgenen Ratten.**

| **Gruppe** | **Mortalität [%]** | **Signifikanz versus Grupp A** |
|---|---|---|
| Gruppe A | 40 | |
| Gruppe B | 20 | |
| Gruppe C | 13 | |
| Gruppe D | 0 | ** |

Parallel zu der kompletten Verhinderung der kardiovaskulären und kardio-renalen Mortalität verbesserten sich auch andere Parameter für Herz-, Herz-Kreislauf und Nierenfunktion. Diese wurden z.B. durch die Bestimmung der Proteinausscheidung über die Niere oder über die BNP-Produktion (Brain Natriuretic Peptide) im Herzen durch Bestimmung der Plasma BNP Spiegel quantifiziert.

### Proteinurie; Protein/Kreatinin Quotient im Urin:

Beim Patienten wird häufig für die Bestimmung des Nierenschadens die Proteinausscheidung in der Niere - die bei Patienten deutlich gesteigert ist - herangezogen. Hierbei wird der Quotient aus dem im Urin ausgeschiedenen Protein und dem im Urin ausgeschiedenen Kreatinin, der sogenannte Protein/Kreatinin Quotient bestimmt, der als quantitatives Maß für den Nierenschaden herangezogen werden kann. Auch in den durchgeführten Tierexperimenten wurde z.B. die Proteniurie, gemessen als Protein/Kreatininquotient im Urin, unter der Behandlung mit Finerenone (10 mg/kg od) alleine bzw. mit dem sGC Stimulator entsprechend der Verbindung der Formel (X) (0.3 mg/kg od) alleine, bereits signifikant um 58% bzw. 51% gesenkt. Auch hier führte aber die Kombination von Finerenone (10 mg/kg od) + Verbindung der Formel (X) (0.3 mg/kg od) zu einer deutlich stärkeren, hochsignifikanten Senkung der Proteinurie insgesamt um 73% (Tabelle 3):

**Tabelle 3: Proteinurie (in % Senkung von Placebo) beim Abschluss der Studie in L-NAME behandelten Renin transgenen Ratten. Daten als Mittelwert ± SEM; */**/***/**** = signifikant mit p<0.05/0.01/0.001/0.0001 (einseitiger ANOVA + post hoc Analyse).**

| **Gruppe** | **Protein/Kreatin Quotient - % von Gruppe A** | **Signifikanz versus Grupp A** |
|---|---|---|
| Gruppe A | +/-0 +15 | |
| Gruppe B | - 58 + 16 | * |
| Gruppe C | - 51 + 20 | * |
| Gruppe D | - 73 + 2 | ** |

## Patentansprüche

1. Kombinationen, die den sGC Stimulator Vericiguat (Methyl-{4,6-diamin-2- [5-fluor-1-(2-fluorbenzyl)-1H-pyrazolo[3,4-b]pyridin-3-yl]pyrimidin-5-yl}carbamat der Formel (X) 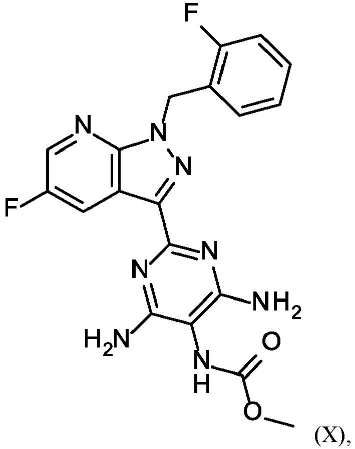 und (*S*)-4-(4-Cyano-2-methoxyphenyl)-5-ethoxy-2,8-dimethyl-1,4-dihydro-1,6-naphthyridin-3-carboxamid der Formel (IV) 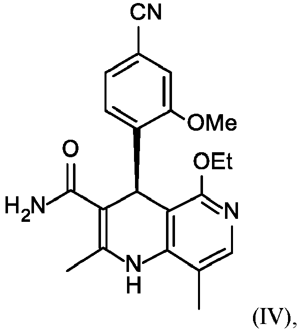 enthält.

2. Kombination gemäß Anspruch 1 zur Behandlung und/oder Prophylaxe von Krankheiten.

3. Kombination gemäß Anspruch 1 zur Behandlung und/oder Prophylaxe von Herz-, Herz-Kreislauf-Erkrankungen, Nieren-, kardio-renalen Erkrankungen, Lungen- und kardio-pulmonalen Erkrankungen, sowie zur Behandlung und/oder Prophylaxe von fibrotischen Erkrankungen sowie von Diabetes und diabetischer und nicht-diabetischer Retinopathie, zur Behandlung und/oder Prophylaxe von Herzinsuffizienz mit erhaltener Ejektionsfraktion oder Herzinsuffizienz mit reduzierter Ejektionsfraktion, Behandlung und/oder Prophylaxe von Vorhofflimmern, Schlaganfall oder Atherosklerose, renalen und kardio-renalen Erkrankungen, chronisches Nierenversagen oder diabetische Nephropathie, zur Behandlung und/oder Prophylaxe von Bluthochdruck, Herzinsuffizienz (akut und chronisch), dekompensierter Herzinsuffizienz, linksventrikulärer Dysfunktion, hypertrophischer Kardiomyopathie, diabetischer Kardiomyopathie, supraventrikulären und ventrikulären Arrythmien, Vorhofflimmern, Vorhofflattern, schädlichem Gefäßumbau, Herzinfarkt und Folgeerscheinungen davon, Atherosklerose, Angina (instabil oder stabil), Niereninsuffizienz (diabetisch und nichtdiabetisch), Herzinsuffizienz, Angina pektoris, Diabetes, sekundärem Hyperaldosteronismus, primärer und sekundärer pulmonaler Hypertonie, Glomerulonephritis, Sklerodermie und Systemischer Sklerose, glomulerärer Sklerose, Proteinurie als Folge einer primären Nierenkrankheit, renaler vaskulärer Hypertonie, diabetischer und nicht-diabetischer Retinopathie, Migräne, periphärer Gefäßkrankheit, Raynaud-Krankheit, luminaler Hyperplasie, kognitiver Dysfunktion, Glaukom und Schlaganfall.

4. Arzneimittel enthaltend mindestens eine Kombination gemäß Anspruch 1 in Kombination mit einem inerten, nicht-toxischen, pharmazeutisch geeigneten Hilfsstoff.

5. Arzneimittel enthaltend mindestens eine Kombination gemäß Anspruch 1, in Kombination mit einem oder mehreren weiteren Wirkstoffen ausgewählt aus der Gruppe bestehend aus ACE-Inhibitoren, Angiotensin Rezeptor Blockern, Kombination von Angiotensin Rezeptor Blockern und NEP-Inhibitoren (ARNIs), Antidiabetika, Beta-Blocker, Acetylsalicylsäure, Diuretika, I_{f}-Kanal-Blocker (Ivabradin), Calcium-Antagonisten, Statine, Digitalis (Digoxin)-Derivate, Calcium-Sensitizer, Nitrate sowie Antithrombotika.

6. Arzneimittel gemäß Anspruch 4 oder 5 zur Behandlung und/oder Prophylaxe von Herz-Kreislauf-Erkrankungen, renalen Erkrankungen, Lungenerkrankungen, sowie zur Behandlung und/oder Prophylaxe von fibrotischen Erkrankungen sowie von Diabetes und diabetischer und nicht-diabetischer Retinopathie.

7. Kombinationen gemäß Anspruch 1 und 4 zur Behandlung und/oder Prophylaxe von Krankheiten, wobei 10 bis 40 mg Finerenone verabreicht werden.

8. Kombinationen gemäß Anspruch 1, wobei bei oraler Applikation von Finerenone 1 bis 100 mg od oder 2.5 bis 50 mg od oder 10 bis 40 mg od verabreicht werden.

9. Kombination gemäß Anspruch 1, wobei bei oraler Applikation von Vericiguat gemäß der Verbindung der Formel (X) 0.5 bis 50 mg od oder 1 bis 15 mg od oder 1.25 bis 10 mg od verabreicht werden.

10. Ein Kit umfassend eine pharmazeutische Zusammensetzung umfassend Finerenone und die Verbindung der Formel (X).

11. Kombinationen nach Anspruch 1, wobei die Komponenten zusammen oder nacheinander oder getrennt in einer kombinierten Einheitsdosierungsform oder in zwei getrennten Einheitsdosierungsformen verabreicht werden.

12. Kombination nach Anspruch 1, wobei die Komponenten der Kombination in getrennten Einheitsdosierungsformen verabreicht werden, wobei die Einheitsform eine Kapsel oder Tablette ist.

13. Kombination nach Anspruch 1, wobei die Kombination eine Einheitsdosierungsform und die Einheitsdosierungsform eine fixierte Kombination ist.

14. Kombination nach Anspruch 1, wobei eine therapeutisch wirksame Menge jeder Komponente der Kombination simultan oder sequenziell in jeder Reihenfolge verabreicht wird.

15. Eine pharmazeutische Formulierung enthaltend eine Kombination nach Anspruch 1, sowie die Salze, Solvate und Solvate der Salze der zu kombinierenden Komponenten.

## Claims

1. Combinations comprising the sGC stimulator vericiguat (methyl {4,6-diamino-2-[5-fluoro-1-(2-fluorobenzyl)-1H-pyrazolo[3,4-b]pyridin-3-yl]pyrimidin-5-yl}carbamate) of the formula (X) 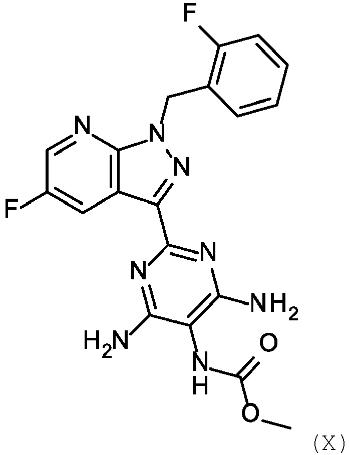 and (S)-4-(4-cyano-2-methoxyphenyl)-5-ethoxy-2,8-dimethyl-1,4-dihydro-1,6-naphthyridine-3-carboxamide of the formula (IV)

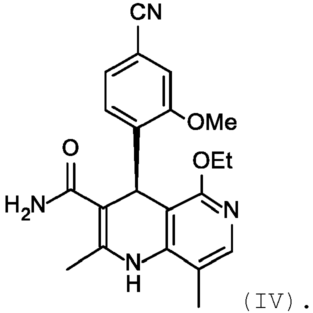

2. Combination according to Claim 1 for the treatment and/or prophylaxis of diseases.

3. Combination according to Claim 1 for the treatment and/or prophylaxis of cardiac disorders, cardiovascular disorders, renal disorders, cardiorenal disorders, pulmonary and cardiopulmonary disorders, and also for the treatment and/or prophylaxis of fibrotic disorders, and also of diabetes and diabetic and non-diabetic retinopathy, for the treatment and/or prophylaxis of heart failure with maintained ejection fraction or heart failure with reduced ejection fraction, treatment and/or prophylaxis of atrial fibrillation, stroke or atherosclerosis, renal and cardiorenal disorders, chronic kidney failure or diabetic nephropathy, for the treatment and/or prophylaxis of hypertension, heart failure (acute and chronic), decompensated heart failure, left ventricular dysfunction, hypertrophic cardiomyopathy, diabetic cardiomyopathy, supraventricular and ventricular arrythmias, atrial fibrillation, atrial flutter, detrimental vascular remodelling, myocardial infarction and sequelae thereof, atherosclerosis, angina (unstable or stable), renal failure (diabetic and non-diabetic), heart failure, angina pectoris, diabetes, secondary hyperaldosteronism, primary and secondary pulmonary hypertension, glomerulonephritis, scleroderma and systemic sclerosis, glomerular sclerosis, proteinuria as sequela of a primary kidney disease, renal vascular hypertension, diabetic and non-diabetic retinopathy, migraine, peripheral vascular disease, Raynaud disease, luminal hyperplasia, cognitive dysfunction, glaucoma and stroke.

4. Medicament comprising at least one combination according to Claim 1 in combination with an inert, nontoxic, pharmaceutically suitable auxiliary.

5. Medicament comprising at least one combination according to Claim 1, in combination with one or more further active compounds selected from the group consisting of ACE inhibitors, angiotensin receptor blockers, combinations of angiotensin receptor blockers and NEP inhibitors (ARNIs), antidiabetics, betablockers, acetylsalicylic acid, diuretics, I_{f} channel blockers (ivabradin), calcium antagonists, statins, digitalis (digoxin) derivatives, calcium sensitizers, nitrates and antithrombotics.

6. Medicament according to Claim 4 or 5 for the treatment and/or prophylaxis of cardiovascular disorders, renal disorders, lung disorders, and also for the treatment and/or prophylaxis of fibrotic disorders, and also of diabetes and diabetic and non-diabetic retinopathy.

7. Combinations according to Claims 1 and 4 for the treatment and/or prophylaxis of diseases, where 10 to 40 mg of finerenone are administered.

8. Combinations according to Claim 1, wherein, in the case of oral administration of finerenone, 1 to 100 mg od or 2.5 to 50 mg od or 10 to 40 mg od are administered.

9. Combination according to Claim 1, wherein, in the case of oral administration of vericiguat as per the compound of the formula (X), 0.5 to 50 mg od or 1 to 15 mg od or 1.25 to 10 mg od are administered.

10. A kit comprising a pharmaceutical composition comprising finerenone and the compound of the formula (X) .

11. Combinations according to Claim 1, wherein the components are administered together or successively or separately in a combined unit dosage form or in two separate unit dosage forms.

12. Combination according to Claim 1, wherein the components of the combination are administered in separate unit dosage forms, wherein the unit form is a capsule or tablet.

13. Combination according to Claim 1, wherein the combination is a unit dosage form and the unit dosage form is a fixed combination.

14. Combination according to Claim 1, wherein a therapeutically effective amount of each component of the combination is administered simultaneously or sequentially in any sequence.

15. A pharmaceutical formulation comprising a combination according to Claim 1, and also the salts, solvates and solvates of the salts of the components to be combined.

## Revendications

1. Combinaisons qui contiennent le stimulateur de la sGC Vericiguat {4,6-diamine-2-[5-fluoro-1-(2-fluorobenzyl)-1H-pyrazolo[3,4-b]pyridin-3-yl]pyrimidin-5-yl}carbamate de méthyle de formule (X) 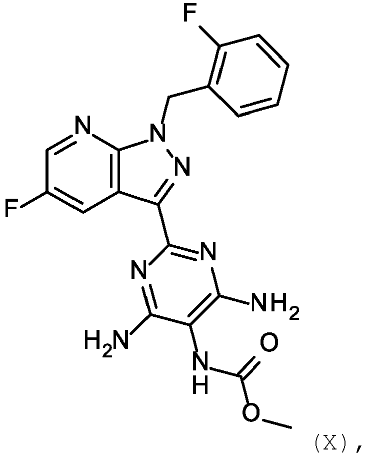 et du (S)-4-(4-cyano-2-méthoxyphényl)-5-éthoxy-2,8-diméthyl-1,4-dihydro-1,6-naphtyridine-3-carboxamide de formule (IV)

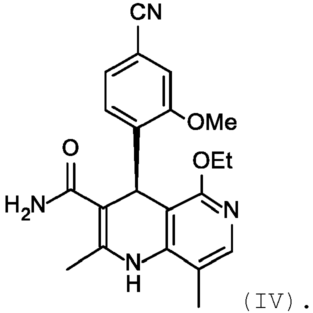

2. Combinaison selon la revendication 1 destinée au traitement et/ou à la prophylaxie de maladies.

3. Combinaison selon la revendication 1 destinée au traitement et/ou à la prophylaxie de maladies cardiaques et cardiovasculaires, de maladies rénales et cardio-rénales, de maladies pulmonaires et cardiopulmonaires, ainsi qu'au traitement et/ou à la prophylaxie de maladies fibrotiques ainsi que du diabète et de la rétinopathie diabétique et non diabétique, au traitement et/ou à la prophylaxie de l'insuffisance cardiaque avec fraction d'éjection préservée ou de l'insuffisance cardiaque avec fraction d'éjection réduite, au traitement et/ou à la prophylaxie de la fibrillation auriculaire, d'un accident vasculaire cérébral ou de l'athérosclérose, de maladies rénales et cardio-rénales, de l'insuffisance rénale chronique ou de la néphropathie diabétique, au traitement et/ou à la prophylaxie de l'hypertension, de l'insuffisance cardiaque (aiguë et chronique), de l'insuffisance cardiaque décompensée, du dysfonctionnement ventriculaire gauche, de la cardiomyopathie hypertrophique, de la cardiomyopathie diabétique, des arythmies supraventriculaires et ventriculaires, de la fibrillation auriculaire, du flutter auriculaire, du remodelage vasculaire néfaste, de l'infarctus du myocarde et de ses conséquences, de l'athérosclérose, de l'angor (instable ou stable), de l'insuffisance rénale (diabétique et non diabétique), de l'insuffisance cardiaque, de l'angine de poitrine, du diabète, de l'hyperaldostéronisme secondaire, de l'hypertonie pulmonaire primaire et secondaire, de la glomérulonéphrite, de la sclérodermie et de la sclérose systémique, de la sclérose glomérulaire, de la protéinurie en tant que conséquence d'une maladie rénale primaire, de l'hypertonie vasculaire rénale, de la rétinopathie diabétique et non diabétique, de la migraine, d'une maladie vasculaire périphérique, de la maladie de Raynaud, de l'hyperplasie luminale, du dysfonctionnement cognitif, du glaucome et d'un accident vasculaire cérébral.

4. Médicament contenant au moins une combinaison selon la revendication 1 en combinaison avec un adjuvant inerte, non toxique, pharmaceutiquement approprié.

5. Médicament contenant au moins une combinaison selon la revendication 1, en combinaison avec un ou plusieurs autres principes actifs choisis dans le groupe constitué par les inhibiteurs de l'ECA, les bloqueurs des récepteurs de l'angiotensine, la combinaison de bloqueurs des récepteurs de l'angiotensine et d'inhibiteurs de NEP (ARNI), les antidiabétiques, les bêta-bloquants, l'acide acétylsalicylique, des diurétiques, les bloqueurs du courant I_{f} (ivabradine), les antagonistes du calcium, les statines, les dérivés de digitalis (digoxine), les sensibilisateurs calciques, les nitrates ainsi que les antithrombotiques.

6. Médicament selon la revendication 4 ou 5 destiné au traitement et/ou à la prophylaxie de maladies cardiovasculaires, de maladies rénales, de maladies pulmonaires, ainsi qu'au traitement et/ou à la prophylaxie de maladies fibrotiques ainsi que du diabète et de la rétinopathie diabétique et non diabétique.

7. Combinaisons selon la revendication 1 et 4 destinées au traitement et/ou à la prophylaxie de maladies, 10 à 40 mg de finérénone étant administrés.

8. Combinaisons selon la revendication 1, 1 à 100 mg od ou 2,5 à 50 mg od ou 10 à 40 mg od étant administrés en cas d'administration par voie orale de finérénone.

9. Combinaison selon la revendication 1, 0,5 à 50 mg od ou 1 à 15 mg od ou 1,25 à 10 mg od étant administrés en cas d'administration de Vericiguat selon le composé de formule (X).

10. Kit comprenant une composition pharmaceutique comprenant de la finérénone et le composé de formule (X) .

11. Combinaisons selon la revendication 1, les composants étant administrés ensemble on consécutivement ou séparément dans une forme de dosage unitaire combinée ou dans deux formes de dosage unitaires séparées.

12. Combinaison selon la revendication 1, les composants de la combinaison étant administrés dans des formes de dosage unitaires séparées, la forme unitaire étant une capsule ou un comprimé.

13. Combinaison selon la revendication 1, la combinaison étant une forme de dosage unitaire et la forme de dosage unitaire étant une combinaison fixe.

14. Combinaison selon la revendication 1, une quantité thérapeutiquement active de chaque composant de la combinaison étant administrée simultanément ou séquentiellement dans n'importe quel ordre.

15. Formulation pharmaceutique contenant une combinaison selon la revendication 1, ainsi que les sels, les solvates et les solvates des sels des composants à combiner.
